# EUROPÄISCHE PATENTSCHRIFT

(11) **EP 1 159 923 B1**
(45) Veröffentlichungstag und Bekanntmachung des Hinweises auf die Patenterteilung: **01.06.2005**
(21) Anmeldenummer: 00250168.2
(22) Anmeldetag: 31.05.2000
(51) Int. Cl.: A61B 17/16

(54) **Chirurgischer Werkzeug-Set**
Surgical tool-set
Ensemble d'outils chirurgical

(43) Veröffentlichungstag der Anmeldung: 05.12.2001
(73) Patentinhaber: Reich, Karl Fritz, 14052 Berlin (DE)
(72) Erfinder: Reich, Karl Fritz, 14052 Berlin (DE)

(56) Entgegenhaltungen:
- DE-C- 19 839 950
- US-A- 5 772 664
- US-A- 5 921 987
- PATENT ABSTRACTS OF JAPAN vol. 2000, no. 03, 30. März 2000 (2000-03-30) & JP 11 347039 A (KO HIDETAKA), 21. Dezember 1999 (1999-12-21)

## Beschreibung

Die Erfindung betrifft einen Hohlschneider, insbesondere ein Werkzeug-Set zum Gewinnen eines gebohrten Knochenkanals als Kompaktknochen (Knochenzylinder).

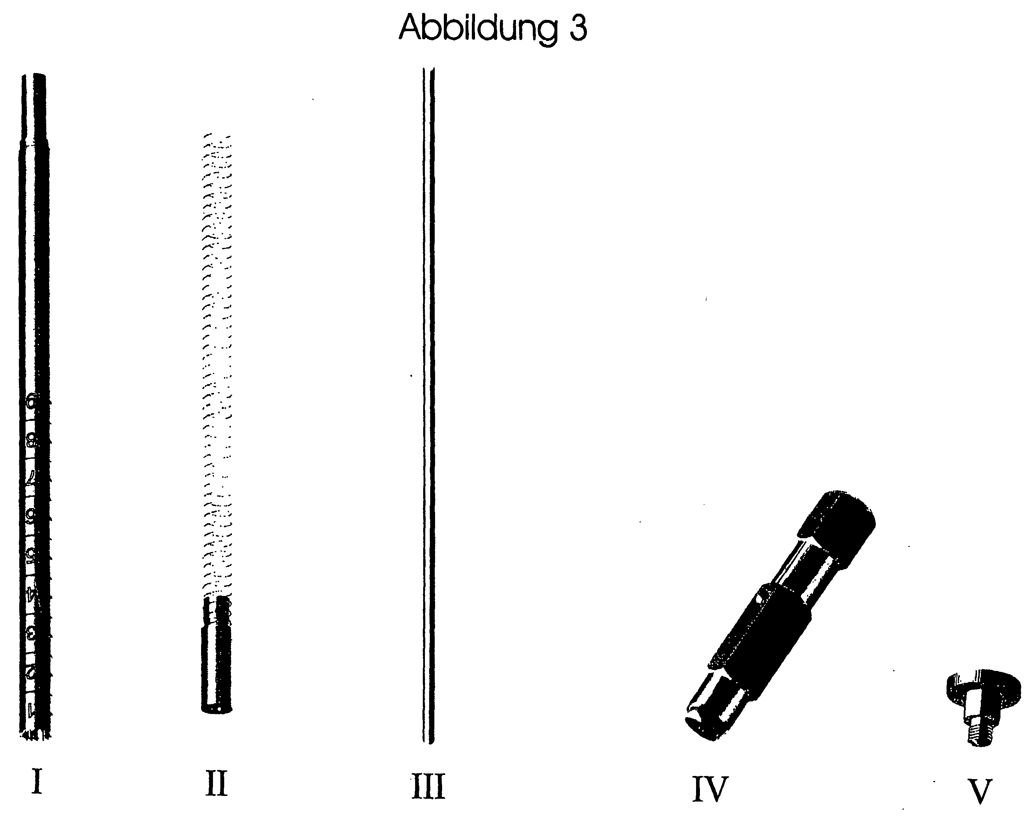

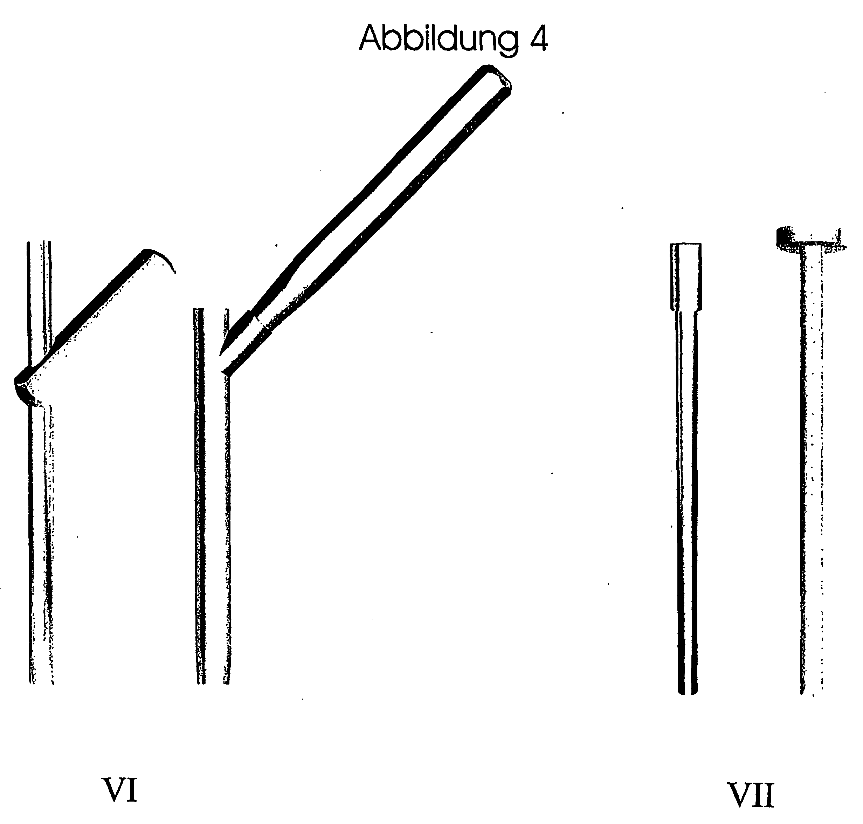

Bei der hier vorgestellten Erfindung ist das Kernstück der Hohlfräser Abb. 3/I und der Führungszylinder Abb. 3/II und der Duce Abb. 4. Hohlschneider werden in der Holzwirtschaft an vielen Stellen eingesetzt. Auch in der Metallindustrie werden Hohlschneider als Hohlfräser verwendet. In der Zahnmedizin, Tiermedizin und auch in der Humanmedizin gibt es Anwendungen. Das zielgenaue Anwenden (Bohren + Fräsen) und das Trennen der in den Hohlschneider gelangten Materie von der umliegenden Materie bereitet bei den bekannten Werkzeugen Schwierigkeiten. Der hier vorgestellte Hohlschneider ermöglicht es, bisher nicht durchführbare Arbeitsgänge unter bestimmten Randbedingungen und mit vorher erwartetem Ergebnis auszuführen. Bei Operationen am menschlichen Stützgerüst und an den Extremitäten und am tierischen Skelett ist es nötig, Bohrungen einzubringen. Die Operationstechnik ist heute Routine. Die dazu nötigen Bohrwerkzeuge werden von der Industrie angeboten. Ziel und Wunsch aller Chirurgen ist es, den zerspanten Bohrkanal als Knochenzylinder zu gewinnen und diesen gewonnenen Knochenzylinder während der Operation im Körper zu "verbauen".

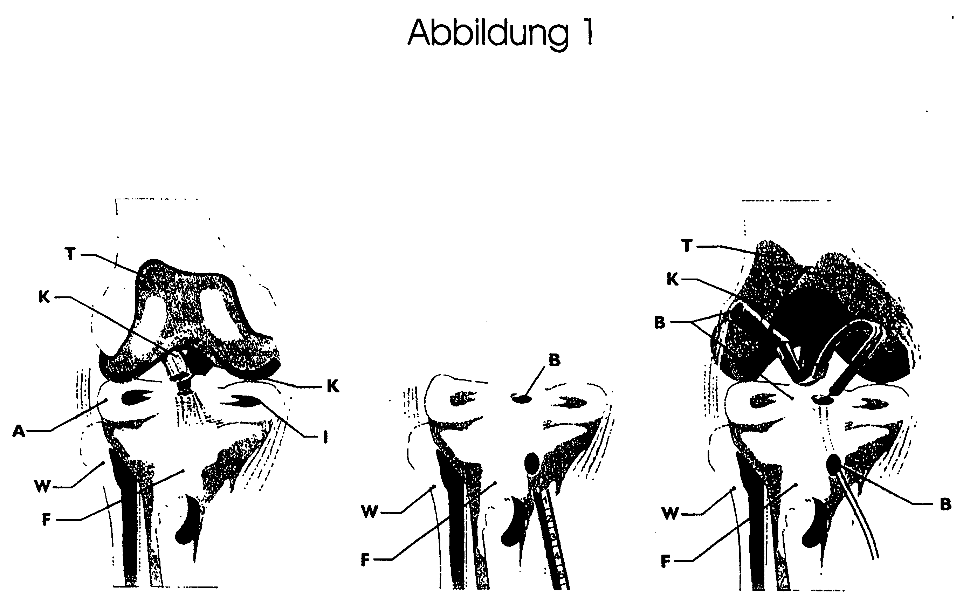

Ein Beispiel: eine der häufigsten Sportverletzungen ist der Riss des Kreuzbandes (Abb. 1).

| Erläuterung: | |
|---|---|
| Oberschenkelknochen | T |
| Außenmeniskus | A |
| Wadenbein | W |
| Schienbein | F |
| Kreuzband | K |
| Innenmeniskus | I |
| Oberschenkelknochen | T |
| Kreuzband | K |
| Bohrkanal | B |

Bei dieser Verletzung müssen in Tibia und/ oder Femur Bohrkanäle eingebracht werden. Die Bohrkanäle haben einen Durchmesser von 6 - 12 mm. Das Ziel ist, diesen durch den Spiralbohrer jetzt zerspanten Knochenzylinder als Kompaktknochen zu gewinnen und während der Operation zu verarbeiten.

### Stand der Technik

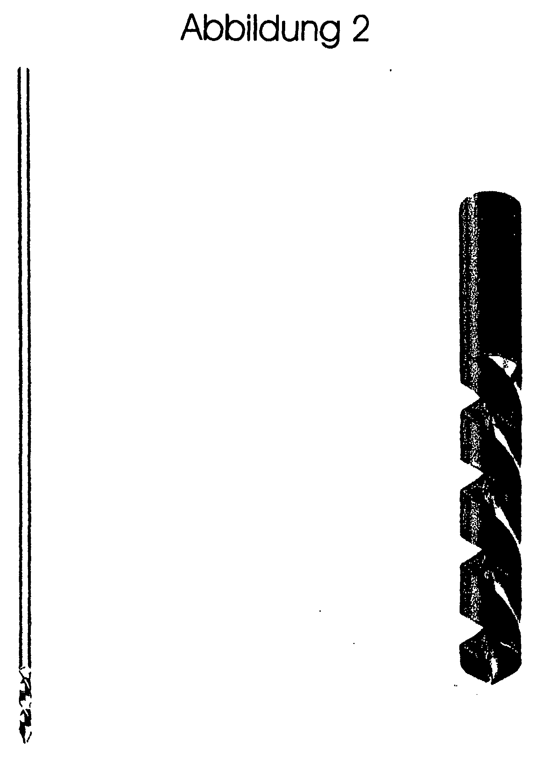

Um einen Bohrkanal herzustellen, wird z.Zt. mit einem käuflich zu erwerbenden Set gearbeitet. Das Set besteht aus dem Führungsbohrer (Trokar) und Spiralbohrern mit Kühlkanal (Abb. 2). Der Führungsbohrer (Trokar) ist ca. 300 mm lang und hat einen Durchmesser von 2,5 mm.
Die Spiralbohrer können 6,7,8,9,10,11, 12 mm ⌀ haben.
Der Kühlkanal in den Spiralbohrern hat immer einen Durchmesser von 2,5 mm. Das Arbeiten mit diesem Set wird im Hörsaal vor Medizinstudenten bereits an der Universität gelehrt. Zuerst setzt der Chirurg einen Führungsbohrer (Trokar) von z.B. 2,5 mm ⌀. Dieser Führungsbohrer bleibt am Ort verankert. Auf diesen Führungsbohrer wird ein Spezialbohrer mit Kühlkanal geschoben, z.B. mit einem Außendurchmesser von 8 mm und einem Innendurchmesser (Kühlkanal) von 2,5 mm. Der 8 mm ⌀ messende Bohrer sitzt fest gespannt in einem Futter. Das Futter sitzt auf dem rotierenden Flansch einer Bohrmaschine. Der Chirurg bohrt mit dieser Kombination, geführt von dem festsitzenden Führungsbohrer (Trokar) (2,5 mm ⌀) in die Knochenmasse.Nach dem Zurückziehen des Spiralbohrers und Entfernen des Führungsbohrers (Trokar) ist ein Loch (ein Kanal) entstanden. Die bei diesem Eingriff entstehenden Knochenspäne werden weggespült.

Dokument DE 198 39 950 C1 offenbart ein Instrument zur Entnahme und zum Einsetzen von Knorpel-Knochen-Blöcken, bestehend aus einem kreiszylindrischen, um seine Längsachse drehbaren Schneidwerkzeug, dessen Stirnkante eine Schneide bildet, und einer das Schneidwerkzeug umgebenden Hülse mit drei in Umfangsrichtung gegeneinander versetzt an ihrer Stirnkante achsparallel angeordneten Stützfüßen, wobei die Hülse auf dem Schneidwerkzeug längsverschieblich gelagert ist.

Mit der Erfindung des Werkzeug-Sets gemäß Anspruch 1 ist es möglich, den gebohrten Knochenkanal als Kompaktknochen (Knochenzylinder) zu gewinnen. Dieser gewonnene Knochenzylinder wird während der Operation benutzt, um Defekte auszugleichen. Der Knochenzylinder kann auch in den gebohrten Knochenkanal eingesetzt werden. Für das Einsetzen wird der zum Operations-Set gehörende Duce benutzt. Das Werkzeug-Set besteht aus Besteck (Abb.3).
I. Hohlfräser von 6,7,8,9,10,11,12 mm ⌀
II. Führungszylinder 5,6,7,8,9,10,11 mm ⌀
III. Ausstoßer 3 mm ⌀
IV. Handgriff
V. Feststellschraube 2 Stück Duce (Abb. 4)
VI. Duce-Führung 6,7,8,9,10,11,12 mm ⌀
VII Stößel 5,6,7,8,9,10,11 mm ⌀

Die I. Hohlfräser, II. Führungszylinder, VI. Duce-Führung, VII. Stößel können auch andere Durchmesser haben. Die Anwendung des Werkzeug-Sets ist für den geschickten Chirurgen einfach.Wie mit den Spiralbohrern im jetzigen Verfahren wird auch bei dieser Anwendung zuerst ein(der) Führungsbohrer (Trokar) (2,5 mm ⌀) gesetzt Abb. 2.

Auf den Führungsbohrer (Trokar) Abb. 2 wird der Führungszylinder Abb. 3/II mit Druckfeder geschoben. Über den Führungsbohrer und den aufgeschobenen Führungszylinder mit Druckfeder wird der Hohlfräser Abb. 3/I geschoben. Der Schaft des Hohlfräsers Abb. 3/I sitzt fest in einem Futter. Das Futter sitzt auf dem rotierenden Flansch einer (der) Bohrmaschine. Mit der rotierenden Welle der Bohrmaschine und damit dem rotierenden Hohlfräser Abb. 3/I, geführt über den Führungszylinder Abb. 3/II, der auf dem festsitzenden Führungsbohrer (Trokar) gleitet (Abb. 2),setzt der Chirurg an der Knochenmasse an.
Der Hohlfräser Abb. 3/I dringt in die Knochenmasse. An der auf dem Hohlfräser angebrachten Markierung Abb. 3/I sieht der Chirurg die Eindringtiefe des Hohlfräsers. In der Knochenmasse befinden sich Führungsbohrer (Trokar) Abb. 2, der Hohlfräser Abb. 3/I, der Führungszylinder Abb. 3/II mit Druckfeder. Diese Kombination wird aus der Knochenmasse entfernt. Der Ausstoßer Abb. 3/III ist in den Handgriff Abb. 3/IV eingesetzt und mit 2 Feststellschrauben Abb. 3/V verriegelt. Mit diesem Ausstoßer Abb. 3/III, der im Hohlfräser Abb. 3/I auf den Führungszylinder Abb. 3/II aufsetzt, wird die Knochenmasse ausgestoßen. Der so gewonnene Knochenzylinder wird in die Duce-Führung Abb. 4/VI eingeschoben. Der Stößel Abb. 4/VII des Duce wird in die Duce-Führung geschoben. Der Duce wird an den gebohrten Knochenkanal oder an einer anderen Stelle, wo ein Knochendefekt ist, angesetzt. Der Chirurg schlägt mit dem Hammer den Abb. 4/VII Stössel des Duce und treibt den Knochen aus der Duce-Führung Abb. 4/VI und von dort in den Knochenkanal.

## Patentansprüche

1. Werkzeug-Set bestehend aus einem Führungsbohrer, einem Führungszylinder (3/II), einer Druckfeder, einem Hohlfräser (3/I) und einem Duce bestehend aus einer Duce-Führung (4/VI) und einem Stößel (4/VII), **dadurch gekennzeichnet, daß** auf den in eine Masse eingebrachten Führungsbohrer der Führungszylinder mit Durchgangsbohrung und über beide der in das Futter einer rotierenden Bohrmaschine gespannte Hohlfräser geschoben werden kann, wobei die Schneide des Hohlfräsers eine Sägezahnschneide haben kann.

2. Werkzeug-Set nach Patentanspruch 1, **dadurch gekennzeichnet, daß** auf den Führungsbohre der Führungszylinder, die Druckfeder und über die drei Elemente der in das Futter einer rotierenden Bohreinheit gespannte Hohlfräser (3/I) geschoben werden kann.

3. Werkzeug-Set nach Patentanspruch 2, **dadurch gekennzeichnet, daß** der Führungszylinder (3/II) mit der Druckfeder durch Drucksitz, Schweissen, Löten, Kleben, Reiben oder durch eine andere Verbindung direkt oder über einen extra Ansatz verbunden ist.

4. Werkzeug-Set nach Patentanspruch 1 oder 2, **dadurch gekennzeichnet, daß** der Führungszylinder an der Schneide des Hohlfräsers (3/I) mit einer Auflage aus Titan, Diamantstaub oder anderen harten Materialien versehen ist.

5. Werkzeug-Set nach Patentanspruch 1 oder 2, **dadurch gekennzeichnet, daß** der Mantel des Hohlfräsers mit einer Auflage, bestehend aus Titan, Schwarzchrom oder anderen Materialien/Auflagen versehen ist.

6. Werkzeug-Set nach Patentanspruch 1, 2, 4 oder 5, **dadurch gekennzeichnet, daß** der Hohlfräser (3/I) mit einer Markierung versehen ist, die dem Benutzer anzeigt, wie tief der Hohlfräser (3/I) in die Masse eindringt.

7. Werkzeug-Set nach Patentanspruch 1, **dadurch gekennzeichnet, daß** der Innendurchmesser der Duce-Führung (4/VI) und die Länge der Duce-Führung (4/VI) so dimensioniert sind, daß der gewonnene Knochenzylinder aufgenommen werden kann und außerdem der Stößel (4/VII) in die Bohrung der Duce-Führung (4/VI) über den Knochenzylinder eingeführt werden kann.

8. Werkzeug-Set nach Patentanspruch 1 oder 7, **dadurch gekennzeichnet, daß** die Duce-Führung des Duce (4/VI) mit einem Griff versehen ist, der auch so gestaltet sein kann, daß er abnehmbar ist.

9. Werkzeug-Set nach Patentanspruch 1, 7 oder 8, **dadurch gekennzeichnet, daß** die Duce-Führung (4/VI) an ihrem Zylinderende mit einer Zahnung versehen ist.

10. Werkzeug-Set nach Patentanspruch 1, 7, 8 oder 9, **dadurch gekennzeichnet, daß** die Duce-Führung (4/VI) an ihrem Zylinderende nach dem Zylinderrand verjüngt ist und dieser Teil der Duce-Führung geschlitzt ist.

## Claims

1. The tool set consists of trocar fig. 2, guide cylinder fig. 3/II, compression spring, hole saw fig. 3/I and duce fig. 4 consisting of duce guide fig. 4/VI and plunger fig. 4/VII **characterized by** the trocar fixed into the mass over which the guide cylinder with linear guide hole is slipped, over which in turn the hole saw, clamped into the chunk of a rotating drill, is slipped, whereby the hole saw cutting edge can be saw teeth.

2. Patent claim referring to patent claim 1 **characterized by** a combination whereby onto the trocar fig. 2 the guide cylinder, a compression spring and over all three components the hole saw fig. 3/I, clamped into the chunk of a rotating drill, is slipped.

3. Patent claim 3 referring to patent claim 2 **characterized by** the design of the guide cylinder fig. 3/II being connected to the compression spring through clamping, welding, soldering, bonding, friction welding or a different joining technique, directly or through an extra connector.

4. Patent claim 4 referring to patent claim 1 + 2 **characterized by** a further design whereby the cylindrical cutting the cylindrical cutting edge of the hole saw fig. 3/I can be coated with titanium, diamond dust or other hard materials.

5. Patent claim 5 referring to patent claim 1 + 2 **characterized by** further design whereby the sleeve of the hole saw is coated in titanium, black chromium or other materials / coatings.

6. Patent claim 6 referring to patent claim 1 + 2 + 4 +5 **characterized by** the design of the hole saw's fig. 3/I outer sleeve marking, showing the user how far the hole saw fig. 3/I penetrates into the matter.

7. Patent claim 7 referring to patent claim 1 consisting of duce fig. 4 which in turn consists of plunger 4/VII and duce guide 4/VI **characterized** the inner diameter of the duce guide 4/VI and the lengths of the duce guide 4/VI the dimension being such that the harvested bone cylinder can be inserted, as well as the plunger 4/VII into the bore of the duce guide 4/VI on top of the bone cylinder.

8. Patent claim 8 referring to patent claim 1 + 7 **characterized by** the design of the duce guide fig. 4 can also be supplied with a handle fig. 4/VI which can also be designed to be removable.

9. Patent claim 9 referring to patent claim 1 + 7 + 8 **characterized by** the design of the duce guide 4/VI with saw teeth at its cylindrical end.

10. Patent claim 10 referring to patent claim 1 + 7 + 8 + 9 **characterized** a further design visible in fig. 4 of the duce guide 4/VI in which the cylinder end towards it's rim is tapered and that this part of the duce guide has several slits.

## Revendications

1. Set des utils composé de trokar (Fig. 2), cylindre de guidage (Fig. 3/ii), ressort-poussoir, fraise évidée (Fig. 3/i), et duce composé de guidage de duce (Fig 4/vi) et poussoir (Fig. 4/vii) **caractérisé que** sur le trokar dans la substance on pousse le cylindre de guidage avec percage et sur les deux on pousse la fraise évidée, qui est fixé dans la gaine d'une foreuse tournante; le tranchant de la fraise peut être muni par une lame aux dents de la scie.

2. Revendication de brevet selon revendication de brevet n°1 **caractérisé que** dans une autre modèle sur le trokar (Fig. 2) on pousse le cylindre de guidage, un ressort-poussoir et sur les trois éléments on pousse la fraise évidée (Fig 3/i), qui est fixé dans la gaine d'une unité de forage tournante.

3. Revendication n°3 de brevet selon revendication de brevet n°2 **caractérisé que** dans un autre modèle le cylindre de guidage (Fig. 3/ii) est rattaché avec le ressort-poussoir par pression, soudage électrique, soudage à l'autogène, collage, friction ou un autrerattachement direct ou par un embout.

4. Revendication de brevet selon revendication de brevet n°1 + n°2 **caractérisé que** dans un autre modèle le cylindre sur le tranchant de la fraise évidée (Fig. 3/i) est muni de une couche de titane, poussière de diamant, chrome noir ou d'autres matériaux / couches.

5. Revendication de brevet selon revendication de brevet n°1 + n°2 **caractérisé que** dans un autre modèle de la fraise évidée (Fig. 3/1) le manteau de la fraise évidée est muni d'une couche de titane, chrome noir ou d'autres matérieaux / couches.

6. Revendication de brevet selon revendication de brevet n°1 + n°2 + n°4 + n°5 **caractérisé que** dans un autre modèle la fraise évidée est muni d'une marquage que indique au utilisateur la profondeur de la pénétration de la fraise évidée (Fig. 3/i) dans la substance.

7. Revendication de brevet selon la revendication de brevet n°1 être composé du duce (Fig. 4) qui se compose de pilon (Fig. 4/ vii) et du guidage du duce **caractérisé que** le diamètre intérieur du guidage de duce (Fig. 4/vi) et le longueur du guidage du duce (Fig. 4/ vi) est tellement dimensioné, qu' il peut contenir le cylindre d'os et ultérieusement le pilon (Fig. 4 /vii) qui est introduit dans la forage du guidage du duce (Fig. 4/vi) au dessus le cylindre d'os.

8. Revendication de brevet selon revendication de brevet n°1+ n°7 **caractérisé que** dans un autre modèle selon fig. 4 le guidage du duce (Fig. 4/vi) est muni d'un manche tellement fait, qu'il est aussi démontable.

9. Revendication de brevet selon revendication de brevet n°1 + n°7 + n°8 **caractérisé que** dans un autre modèle selon fig. 4 le guidage du duce (Fig. 4/vi) est à son bout de cylindre muni d'une denture.

10. Revendication de brevet selon revendication de brevet n°1+ n°7 + n°8 + n°9 **caractérisé que** dans un autre modèle selon fig. 4 le guidage du duce (Fig. 4/vi) se rétrécit vers le bout du cylindre et que cette partie du guidage du duce est munie d'une incision.
